# EUROPÄISCHE PATENTSCHRIFT

(11) **EP 1 980 192 B1**
(45) Veröffentlichungstag und Bekanntmachung des Hinweises auf die Patenterteilung: **01.07.2015**
(21) Anmeldenummer: 08006720.0
(22) Anmeldetag: 02.04.2008
(51) Int. Cl.: A61L 9/03

(54) **Textiler Überzug für ein Dampferzeugungsgerät**
Textile cover for a steam generation device
Revêtement textile pour un appareil de production de vapeur

(30) Priorität: 11.04.2007 DE 102007017062
(43) Veröffentlichungstag der Anmeldung: 15.10.2008
(73) Patentinhaber: Uyttenboogaard, Eric M. J., 4147 CT Asperen (NL)
(72) Erfinder: Uyttenboogaard, Eric M. J., 4147 CT Asperen (NL)
(74) Vertreter: Schulte, Jens Michael

(56) Entgegenhaltungen:
- WO-A1-01/52714
- WO-A1-97/29178
- WO-A2-2006/049499
- CA-A1- 2 448 886
- CA-A1- 2 451 551
- DE-U1- 20 001 462
- DE-U1- 20 106 432
- DE-U1- 20 111 178
- DE-U1- 29 822 052
- JP-A- 2001 327 449
- KR-Y1- 200 433 539
- US-A1- 2006 000 049
- US-A1- 2007 020 020

## Beschreibung

Die Erfindung betrifft einen textilen Überzug für ein Dampfreinigungsgerät sowie ein Dampferzeugungsgerät für den Haushalt, mit einem Wassertank, einem Auslass, durch den der in dem Gerät erzeugte Wasserdampf austritt, wobei der Auslass mit einem auswechselbaren textilen Überzug zu versehen ist.

Derartige Dampferzeugungsgeräte für den Haushalt erfreuen sich seit einigen Jahren steigender Beliebtheit, weil eine besonders hygienische Form der Reinigung hiermit möglich ist. Gerade Böden und andere glatte Oberflächen lassen sich mit diesen Dampfbesen gut bearbeiten. Der in dem Gerät erzeugte Dampf ist wegen der hohen Temperatur und der effektiven Reinigung besonders wirksam gegen Bakterien und Verschmutzung und das auf umweltschonende Weise. Der textile Überzug nimmt eine große Menge an Schmutz oder auch Feuchtigkeit auf, ist auswechsel- und damit waschbar. Prinzipiell sollten solche Dampfreiniger ohne Zugabe eines Reinigungsmittels in den Wassertank betrieben werden. Der Einsatz eines solchen Mittels bewirkt aber grundsätzlich über den Reinigungseffekt hinaus auch eine Geruchswirkung. So soll nach einer Reinigung in subjektiver wie objektiver Hinsicht eine gesäuberte Fläche oder ein gereinigter Raum möglichst gut riechen, um die entsprechende Wirkung noch zu verstärken.

Insofern stellt sich der vorliegenden Erfindung die Aufgabe, einen textilen Überzug sowie ein Dampferzeugungsgerät für den Haushalt zu schaffen, das über einen optimalen Reinigungseffekt hinaus auch für einen angenehmen Geruch einer behandelten Fläche oder eines gesäuberten Raumes sorgt.

Diese Aufgabe wird durch ein Dampfreinigungsgerät gemäß Anspruch 1 gelöst. Bevorzugte Ausführungsformen sind in den Unteransprüchen 2-11 aufgeführt.

In dem Reinigungsgerät wird Dampf erzeugt, der dann durch einen Auslass austritt. Durch die Positionierung eines Wirkstoffträgers an oder in dem textilen Überzug strömt der Dampf gezielt an dem Wirkstoffträger vorbei und nimmt dabei einen Hauch von Wirkstoff auf, sei es zur Erzielung eines intensiveren Reinigungszwecks oder zur Verbesserung der Luftqualität. Dem Dampf wird damit z.B. eine leicht duftende Note verliehen und über den primären Reinigungseffekt hinaus wird für einen angenehmen Duft der gesäuberten Fläche oder eines gesäuberten Raumes gesorgt.

Dabei ist vorgesehen, dass der Überzug in einem zu dem Auslass korrespondierenden Flächenabschnitt mindestens eine Aufnahme für einen Wirkstoffträger aufweist, der damit seinerseits auswechselbar ist. Der Wirkstoffträger kann damit im Falle der Abnutzung ausgetauscht werden, dem Benutzer ist es darüber hinaus aber auch möglich, die Art, Zusammensetzung oder Intensität der Reiniger und/oder Düfte zu variieren. Indem der Flächenabschnitt in unmittelbarer Nähe des Dampfauslasses positioniert ist, wird der Dampf an dem Wirkstoffträger quasi vorbeigeleitet, sodass der entsprechende Kontakt auch gewährleistet ist. Die Aufnahme ist dabei in der Form in den Überzug integriert, dass der Wirkstoffträger auch über einen längeren Benutzungszeitraum seine dortige Position beibehält.

In einer besonders bevorzugten Ausführungsform der Erfindung ist dies der Fall, wenn die Aufnahme als Tasche ausgebildet ist, zu welcher der Wirkstoffträger wiederum korrespondiert. Dieser wird einfach von der Seite her in die Tasche eingeschoben, kann jederzeit praktisch ohne Aufwand ausgetauscht werden und ist zudem gegen Herausrutschen gesichert. Darüber hinaus ist der Wirkstoffträger in der Form in das Gerät integriert, dass er von außen gar nicht sichtbar ist.

In einer besonders einfachen Form der Erfindung ist die Tasche durch eine Naht mit dem Überzug verbunden, also zweckmäßigerweise an der Innenseite des Überzugs auf diese aufgesetzt und entsprechend befestigt. Die Tasche ist rechteckförmig ausgebildet und weist an einer ihrer kürzeren Seiten eine Öffnung auf, in welche der Wirkstoffträger seitlich eingeschoben werden kann. Damit ist dieser dort auch sicher für den Fall untergebracht, dass sich im Laufe der Benutzung eine Reduzierung seiner Größe einstellt.

Eine alternative Ausführung zu der Tasche sieht vor, dass die Aufnahme durch mindestens einen spannbaren Riemen gebildet ist. Dabei ist der Wirkstoffträger zwischen dem Überzug und dem oder den Riemen eingeklemmt und entsprechend auch sicher in seiner Position gehalten.

Eine weitere Lösung hierfür sieht vor, dass die Aufnahme durch ein separates Befestigungsmittel gebildet ist, dass über ein externes Befestigungsmittel wie eine Klammer oder eine vergleichbare Vorrichtung also der Wirkstoffträger zusammen mit dieser an der Innenseite des Überzugs befestigt wird, ohne dabei natürlich die Funktion des Reinigungsgerätes und die glatte Arbeitsfläche zu beeinträchtigen.

Es wurde bereit darauf hingewiesen, dass es vorteilhaft ist, wenn die Aufnahme an der Innenseite des Überzugs positioniert ist. Dort ist der Wirkstoffträger einerseits geschützt und beeinflusst andererseits weder die Optik noch insbesondere die Funktion des Gerätes.

Das Dampferzeugungsgerät weist neben seinem eigentlichen Korpus mit den notwendigen Aggregaten wie Wassertank, Griff, Steuerung oder einer Erhitzungsvorrichtung einen Fuß auf. Dieser besitzt eine Geometrie, die es ermöglichen soll, das Dampferzeugungsgerät auch an schlecht zugänglichen Stellen einsetzen zu können. Der Fuß dient außerdem zur Aufnahme des Überzugs, da es natürlich ausreicht, wenn der textile Überzug im unmittelbaren Einwirkbereich angeordnet ist. Es ist daher zusätzlich daran gedacht, dass der Auslass in einen gelenkig gelagerten Fuß integriert ist, der zur Aufnahme des Überzugs dient.

Eine weitere besonders bevorzugte Ausführungsform der Erfindung sieht vor, dass der Wirkstoffträger eine Reinigungssubstanz enthält, welche den mit dem Austritt des Wasserdampfs erzielten Säuberungseffekt noch verstärkt. Alternativ oder ergänzend dazu wird vorgeschlagen, dass der Wirkstoffträger einen Duftstoff enthält, um einen entsprechenden Wohlgeruch zu verbreiten.

Es wurde darauf hingewiesen, dass Ausführungen als zweckmäßig betrachtet werden, bei denen die Aufnahme als Tasche oder als spannbare Riemen ausgebildet ist. Ergänzend wird hierzu vorgeschlagen, dass der Wirkstoffträger als Pellet ausgebildet ist, der mit einem entsprechenden Reinigungs- oder Duftwirkstoff versehen ist und seitlich in die Tasche oder unter den Riemen an der Innenseite des textilen Überzuges positioniert wird.

Ein weiterer vorteilhafter Reinigungseffekt wird erzielt, wenn der Überzug aus Mikrofaser hergestellt ist. Dieses Material gewährleistet einerseits eine hohe Aufnahme von Schmutz und Feuchtigkeit und lässt sich andererseits auf besonders einfache Weise wieder reinigen und neu verwerten.

Die Erfindung betrifft außerdem einen auswechselbaren Überzug gemäß Anspruch 12 für ein Dampferzeugungsgerät für den Haushalt, mit einem Wassertank, einem Auslass, durch den der in dem Gerät erzeugte Wasserdampf austritt, wobei der Auslass mit einem auswechselbaren textilen Überzug versehen ist. Letzterer zeichnet sich dadurch aus, dass er im Bereich des Auslasses des Dampferzeugungsgerätes mit einem Wirkstoffträger ausgerüstet ist.

Der Überzug weist eine Tasche oder sonstige Aufnahme für den Wirkstoffträger auf und kann jederzeit nach Entfernung des Wirkstoffträgers gewaschen bzw. beliebig ausgetauscht werden. Durch Positionierung des Wirkstoffträgers in dem Überzug in unmittelbarer Nähe des geräteseitigen Dampfauslasses strömt der Dampf gezielt an dem Wirkstoffträger vorbei und nimmt Wirkstoff zur Erzielung einer intensiveren Reinigung oder zur Verbesserung der Luftqualität auf.

Die Erfindung zeichnet sich insbesondere dadurch aus, dass ein Dampferzeugungsgerät für den Haushalt geschaffen ist, bei dem der erzeugte Dampf im Rahmen seines Austritts so gezielt an einem Wirkstoffträger herbeigeführt wird, dass damit ein zusätzlicher Reinigungs- und/oder Dufteffekt erreicht werden kann. Hierfür ist ein den Wirkstoffträger aufnehmendes Pellet in unmittelbarer Nähe des Dampfauslasses des Gerätes positioniert und dem auswechselbaren textilen Überzug zugeordnet. Zweckmäßigerweise befindet sich hierzu an der Innenseite des Überzugs eine Tasche, in die der Wirkstoffträger seitlich eingeschoben werden kann.

Weitere Einzelheiten und Einzelteile und Vorteile der Erfindung ergeben sich aus der nachfolgenden Beschreibung der zugehörigen Zeichnungen, in denen ein bevorzugtes Ausführungsbeispiel mit den dazu notwendigen Einzelheiten und Einzelteilen dargestellt ist. Es zeigen:
- Figur 1: ein Dampferzeugungsgerät,
- Figur 2: einen textilen Überzug in Ansicht von unten,
- Figur 3: eine Alternative zur Figur 2 und
- Figur 4: eine schematische Darstellung des Funktionsprinzips.

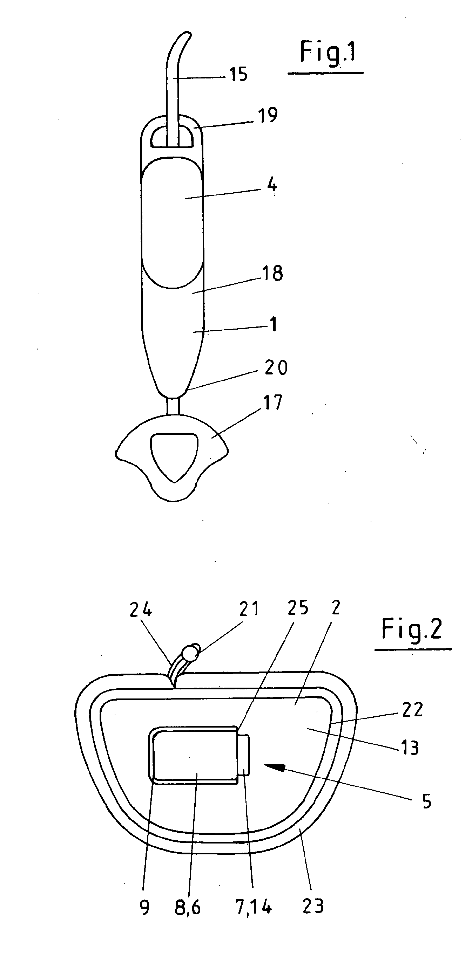

In Figur 1 ist ein handelsübliches Dampferzeugungsgerät 1 dargestellt, dieses umfasst im Bereich seines Korpus 18 Griffe 15 und 19 sowie den Wassertank 4. An der Unterseite 20 des Korpus 18 befindet sich der Fuß 17, der eine geometrische Form aufweist, die es ermöglichen soll, auch schwer zugängliche und enge Stellen beim Säubern erreichen zu können.

Figur 2 zeigt den textilen auswechselbaren Überzug 2 mit Blick auf dessen Innenseite 13. Am äußeren Rand befindet sich eine Umnaht 22, in diesem Bereich wird der korrespondierend zu dem in Figur 1 mit dem Bezugszeichen 17 versehenen Fuß ausgebildete Überzug 2 über den Fuß 17 gestülpt. Mit dem Bezugszeichen 21 ist eine Handhabe bezeichnet, mittels welcher eine den Überzug 2 an seinem seitlichen Rand 23 umlaufende Kordel 24 nach Überzug über den Fuß des Dampferzeugungsgerätes zusammengezogen werden kann, um den Überzug 2 auf dem Fuß zu befestigen. Zentral an der Innenseite 13 des Überzugs 2 im Flächenabschnitt 5 befindet sich die Aufnahme 6 für den Wirkstoffträger 7. Es handelt sich hierbei um ein Pellet 14, das in einer Tasche 8 positioniert und seitlich in eine Öffnung 25 eingeschoben ist. Die Tasche 8 ist über eine Naht 9 mit dem textilen Überzug 2 verbunden. Damit befindet sich der Wirkstoffträger 7 in einem Flächenabschnitt 5 des textilen Überzugs 2, der in unmittelbarer Nähe des Auslasses des Dampferzeugungsgerätes positioniert ist. Es ist damit gewährleistet, dass der aus dem Gerät ausströmende Wasserdampf den mit Reinigungssubstanz und/oder Duftstoff versehenen Wirkstoffträger 7 tatsächlich auch passiert und sich entsprechend anreichert.

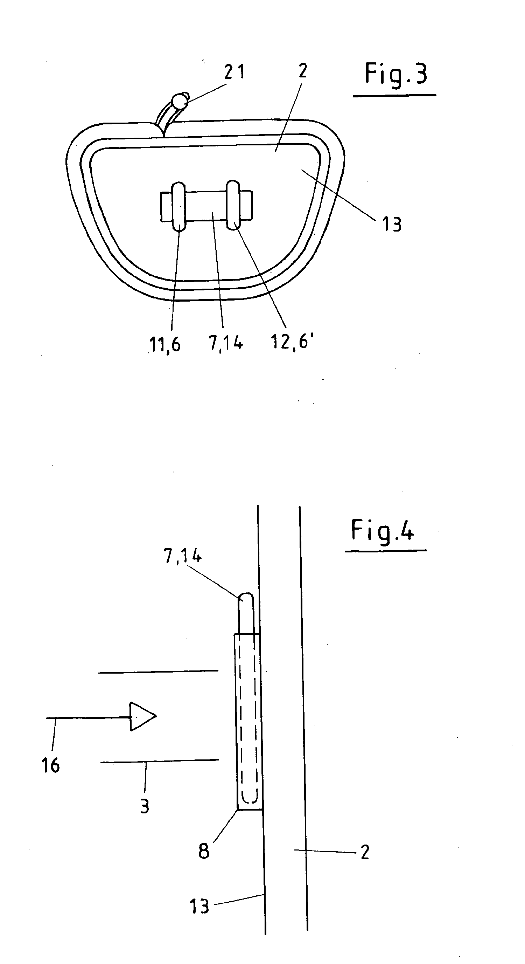

Eine Alternative hierzu zeigt Figur 3. Dort ist der Wirkstoffträger 7 in Form des Pellets 14 gezeigt, wie er unterhalb der beiden als Aufnahme 6, 6' dienenden Riemen 11, 12 eingeklemmt ist und sich damit ebenfalls in dem relevanten Flächenabschnitt 5 des textilen Überzugs 2 befindet, damit der Wasserdampf beim Austritt aus dem Dampferzeugungsgerät um Duftstoff und/oder Reinigungsmittel angereichert wird.

Figur 4 betrifft eine schematische Darstellung des erfindungsgemäßen Prinzips. Dort ist ein Auslass 3 des im Übrigen nicht dargestellten Dampferzeugungsgerätes im Schnitt gezeigt, aus dem der Wasserdampf in Pfeilrichtung 16 austritt. Dabei passiert der Wasserdampf den in der Tasche 8 positionierten Wirkstoffträger 7, hier in Form des Pellets 14 an der Innenseite 13 des textilen Überzugs 2 beim Durchströmen des letzteren und tritt entsprechend angereichert in die Atmosphäre.

## Patentansprüche

1. Dampferzeugungsgerät (1) für den Haushalt, mit einem Wassertank (4), einem Auslass (3), durch den der in dem Gerät (1) erzeugte Wasserdampf austritt, wobei der Auslass (3) mit einem auswechselbaren textilen Überzug (2) versehen ist,
**dadurch gekennzeichnet,**
**dass** der textile Überzug (2) im Bereich des Auslasses (3) des Dampferzeugungsgerätes (1) in einem zu dem Auslass (3) korrespondierenden Flächenabschnitt (5) mindestens eine Aufnahme (6) für einen Wirkstoffträger (7) aufweist.

2. Dampferzeugungsgerät nach Anspruch 1,
**dadurch gekennzeichnet,**
**dass** die Aufnahme (6) als Tasche (8) ausgebildet ist.

3. Dampferzeugungsgerät nach Anspruch 2,
**dadurch gekennzeichnet,**
**dass** die Tasche (8) durch eine Naht (9) mit dem Überzug (2) verbunden ist.

4. Dampferzeugungsgerät nach Anspruch 1,
**dadurch gekennzeichnet,**
**dass** die Aufnahme (6) durch mindestens einen spannbaren Riemen (11, 12) gebildet ist.

5. Dampferzeugungsgerät nach Anspruch 1,
**dadurch gekennzeichnet,**
**dass** die Aufnahme (6) durch ein separates Befestigungsmittel gebildet ist.

6. Dampferzeugungsgerät nach Anspruch 1,
**dadurch gekennzeichnet,**
**dass** die Aufnahme (6) an der Innenseite (13) des Überzugs (2) vorgesehen ist.

7. Dampferzeugungsgerät nach Anspruch 1,
**dadurch gekennzeichnet,**
**dass** der Auslass (3) in einen gelenkig gelagerten Fuß (17) integriert ist, der zur Aufnahme des Überzugs (2) dient.

8. Dampferzeugungsgerät nach Anspruch 1,
**dadurch gekennzeichnet,**
**dass** der Wirkstoffträger (7) eine Reinigungssubstanz enthält.

9. Dampferzeugungsgerät nach Anspruch 1,
**dadurch gekennzeichnet,**
**dass** der Wirkstoffträger (7) einen Duftstoff enthält.

10. Dampferzeugungsgerät nach Anspruch 1,
**dadurch gekennzeichnet,**
**dass** der Wirkstoffträger (7) als Pellet (14) ausgebildet ist.

11. Dampferzeugungsgerät nach Anspruch 1,
**dadurch gekennzeichnet,**
**dass** der Überzug (2) aus Mikrofaser hergestellt ist.

12. Auswechselbarer Überzug (2) zur Reinigung von Flächen mittels eines Dampfreinigungsgeräts (1) für den Haushalt, wobei das Dampfreinigungsgerät (1) mit einem Wassertank (4), einem Auslass (3), durch den der in dem Gerät (1) erzeugte Wasserdampf austritt, wobei der Auslass (3) mit dem auswechselbaren textilen Überzug (2) versehen ist,
**dadurch gekennzeichnet,**
**dass** der textile Überzug (2) im Bereich des Auslasses (3) des Dampferzeugungsgerätes (1) in einem zu dem Auslass (3) korrespondierenden Flächenabschnitt (5) mindestens eine Aufnahme (6) für einen Wirkstoffträger (7) aufweist.

## Claims

1. Steam generating device (1) for the household, with a water tank (4), an outlet (3) through which the steam generated in the device (1) escapes, whereby the outlet (3) is fitted with a replaceable textile cover (2),
**characterised by the fact**
that the textile cover (2) in the area of the outlet (3) of the steam generating device (1) in a section (5) corresponding to the outlet (3) has at least one holder (6) for an active substance carrier (7).

2. Steam generating device in accordance with Claim 1,
**characterised by the fact**
that the holder (6) is designed as a pocket (8).

3. Steam generating device in accordance with Claim 2,
**characterised by the fact**
that the pocket (8) is connected by a seam (9) with the cover (2).

4. Steam generating device in accordance with Claim 1,
**characterised by the fact**
that the holder (6) is formed by at least one clampable strap (11, 12).

5. Steam generating device in accordance with Claim 1,
**characterised by the fact**
that the holder (6) is formed by a separate fastening element.

6. Steam generating device in accordance with Claim 1,
**characterised by the fact**
that the holder (6) is planned on the inside (13) of the cover (2).

7. Steam generating device in accordance with Claim 1,
**characterised by the fact**
that the outlet (3) is integrated in a hinged foot (17) that serves as the holder of the cover (2).

8. Steam generating device in accordance with Claim 1,
**characterised by the fact**
that the active substance carrier (7) contains a cleaning substance.

9. Steam generating device in accordance with Claim 1,
**characterised by the fact**
that the active substance carrier (7) contains a fragrance.

10. Steam generating device in accordance with Claim 1,
**characterised by the fact**
that the active substance carrier (7) is formed as a pellet (14).

11. Steam generating device in accordance with Claim 1,
**characterised by the fact**
that the cover (2) is made of microfibre.

12. Replaceable cover (2) for cleaning surfaces by means of a steam cleaner (1) for the household, whereby the steam cleaner (1) is fitted with a water tank (4), an outlet (3), through which the steam generated in the device (1) escapes, whereby the outlet (3) is fitted with the replaceable textile cover (2),
**characterised by the fact**
that the textile cover (2) in the area of the outlet (3) of the steam generating device (1) in a section (5) corresponding to the outlet (3) has at least one holder (6) for an active substance carrier (7).

## Revendications

1. Appareil de production de vapeur (1) pour le ménage, avec un réservoir d'eau (4), une sortie (3) à travers laquelle sort la vapeur d'eau générée dans l'appareil (1), sachant que la sortie (3) est pourvue d'un revêtement textile (2) interchangeable,
**caractérisé par le fait**
**que** le revêtement textile (2) présente au moins une réception (6) pour un support d'agent actif (7) dans la zone de la sortie (3) de l'appareil de production de vapeur (1), dans une section de surface (5) correspondant à la sortie (3).

2. Appareil de production de vapeur selon la revendication 1,
**caractérisé par le fait**
**que** la réception (6) a été réalisée sous la forme d'une poche (8).

3. Appareil de production de vapeur selon la revendication 2,
**caractérisé par le fait**
**que** la poche (8) est reliée au revêtement (2) au moyen d'une couture (9).

4. Appareil de production de vapeur selon la revendication 1,
**caractérisé par le fait**
**que** la réception (6) est constituée d'au moins une courroie (11, 12) pouvant être tendue.

5. Appareil de production de vapeur selon la revendication 1,
**caractérisé par le fait**
**que** la réception (6) est constituée d'un moyen de fixation séparé.

6. Appareil de production de vapeur selon la revendication 1,
**caractérisé par le fait**
**que** la réception (6) est prévue du côté intérieur (13) du revêtement (2).

7. Appareil de production de vapeur selon la revendication 1,
**caractérisé par le fait**
**que** la sortie (3) est intégrée dans un pied (17) sur palier articulé, qui sert de réception au revêtement (2).

8. Appareil de production de vapeur selon la revendication 1,
**caractérisé par le fait**
**que** le support d'agent actif (7) contient une substance de nettoyage.

9. Appareil de production de vapeur selon la revendication 1,
**caractérisé par le fait**
**que** le support d'agent actif (7) contient un parfum.

10. Appareil de production de vapeur selon la revendication 1,
**caractérisé par le fait**
**que** le support d'agent actif (7) est constitué sous la forme d'un pellet (14).

11. Appareil de production de vapeur selon la revendication 1,
**caractérisé par le fait**
**que** le revêtement (2) est réalisé en microfibres.

12. Revêtement (2) interchangeable destiné au nettoyage de surfaces au moyen d'un appareil de production de vapeur (1) pour le ménage, sachant que l'appareil de nettoyage à la vapeur (1) est pourvu d'un réservoir d'eau (4), d'une sortie (3) à travers laquelle sort la vapeur d'eau générée dans l'appareil (1), sachant que la sortie (3) est pourvue d'un revêtement (2) textile interchangeable,
**caractérisé par le fait**
**que** le revêtement textile (2) présente au moins une réception (6) pour un support d'agent actif (7) dans la zone de la sortie (3) de l'appareil de production de vapeur (1), dans une section de surface (5) correspondant à la sortie (3).
